(19) Europäisches Patentamt
European Patent Office
Office européen des brevets

(11) **EP 2 407 912 B1**

(12) # FASCICULE DE BREVET EUROPEEN

(45) Date de publication et mention
de la délivrance du brevet:
**28.08.2024   Bulletin 2024/35**

(21) Numéro de dépôt: **11172901.8**

(22) Date de dépôt: **06.07.2011**

(51) Classification Internationale des Brevets (IPC):
**G16H 50/70** (2018.01)    **G06F 3/01** (2006.01)
**A61B 5/316** (2021.01)    **A61B 5/369** (2021.01)
**G06F 18/2132** (2023.01)    **A61B 5/00** (2006.01)

(52) Classification Coopérative des Brevets (CPC):
**A61B 5/4064; A61B 5/316; A61B 5/369;
A61B 5/7267; G06F 3/015; G06F 18/2132;**
G06F 2218/12; G16H 50/70

(54) **Procédé et système de classification de signaux neuronaux, et procédé de sélection d'électrodes pour commande neuronale directe**

Verfahren und System zur Klassifikation von neuronalen Signalen und Verfahren zur Auswahl von Elektroden für direkte neuronale Steuerung

Method and system for classifying neuronal signals and method for selecting electrodes for direct neuronal control

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **13.07.2010   FR 1002948**

(43) Date de publication de la demande:
**18.01.2012   Bulletin 2012/03**

(73) Titulaire: **COMMISSARIAT À L'ÉNERGIE ATOMIQUE ET AUX ÉNERGIES ALTERNATIVES
75015 Paris (FR)**

(72) Inventeurs:
• **Barachant, Alexandre
38000 Grenoble (FR)**
• **Bonnet, Stéphane
69003 Lyon (FR)**

(74) Mandataire: **Gevers & Orès
Immeuble le Palatin 2
3 Cours du Triangle
CS 80165
92939 Paris La Défense Cedex (FR)**

(56) Documents cités:
**US-A1- 2007 133 878    US-A1- 2008 063 264**

• **GERSCH W ET AL: "A Kullback Leibler-nearest neighbor rule classification of EEGs: The EEG population screening problem, an anesthesia level EEG classification application", COMPUTERS AND BIOMEDICAL RESEARCH, vol. 13, no. 3, June 1980 (1980-06-01), pages 283 - 296, XP022956406, ISSN: 0010-4809, [retrieved on 19800601], DOI: 10.1016/0010-4809(80)90022-1**
• **KAMOUSI B ET AL: "Classification of motor imagery by means of cortical current density estimation and Von Neumann entropy", JOURNAL OF NEURAL ENGINEERING, vol. 4, no. 2, June 2007 (2007-06-01), pages 17 - 25, XP020123711, ISSN: 1741-2552, DOI: 10.1088/1741-2560/4/2/002**
• **YILI LI ET AL: "EEG signal classification based on a Riemannian distance measure", PROCEEDINGS OF 2009 IEEE TORONTO INTERNATIONAL CONFERENCE SCIENCE AND TECHNOLOGY FOR HUMANITY (TIC-STH 2009), 26-27 SEPTEMBER 2009, TORONTO, ONTARIO, CANADA, 26 September 2009 (2009-09-26), pages 268 - 273, XP031655898, ISBN: 978-1-4244-3877-8**
• **BARBARESCO F ET AL: "Espace Riemannien symétrique et géométrie des espaces de matrices de covariance : équations de diffusion et calculs de médianes", ACTES DU COLLOQUE GRETSI 2009, 8-11 SEPTEMBRE 2009, DIJON, FRANCE, 8 September 2009 (2009-09-08), pages 1 - 4, XP007914443**

EP 2 407 912 B1

- SANNELLI C ET AL: "On optimal channel configurations for SMR-based Brain-Computer Interfaces", BRAIN TOPOGRAPHY, vol. 23, no. 2, June 2010 (2010-06-01), pages 186 - 193, XP055002156, ISSN: 0896-0267, DOI: 10.1007/s10548-010-0135-0
- BARACHANT A: "Filtrage spatial robuste à partir d'un sous-ensemble optimal d'électrodes en BCI EEG", ACTES DU COLLOQUE GRETSI 2009, 8-11 SEPTEMBRE 2009, DIJON, FRANCE, 8 September 2009 (2009-09-08), XP055002259, Retrieved from the Internet <URL:http://documents.irevues.inist.fr/bitstrea m/handle/2042/28933/barachant_554.pdf?seque nce=1> [retrieved on 20110707]
- YILI LI: "Multichannel EEG signal classification on a Riemannian manifold", PHD THESIS, DEPARTMENT OF ELECTRICAL AND COMPUTER ENGINEERING, MCMASTER UNIVERSITY, CANADA, September 2010 (2010-09-01), XP055000797
- BARACHANT A ET AL: "Riemannian geometry applied to BCI classification", LATENT VARIABLE ANALYSIS AND SIGNAL SEPARATION, PROCEEDINGS OF LVA/ICA 2010, 27-30 SEPTEMBER 2010, SAINT-MALO, FRANCE, LECTURE NOTES IN COMPUTER SCIENCE 6365, September 2010 (2010-09-01), pages 629 - 636, XP019153437
- BARACHANT A: "Channel selection procedure using Riemannian distance for BCI applications", PROCEEDINGS OF THE 5TH INTERNATIONAL IEEE EMBS CONFERENCE ON NEURAL ENGINEERING, 27 APRIL - 1 MAY 2011, CANCUN, MEXICO, 27 April 2011 (2011-04-27), pages 348 - 351, XP055002151, Retrieved from the Internet <URL:http://ieeexplore.ieee.org/ielx5/5773344/5 910465/05910558.pdf?tp=&arnumber=5910558&i snumber=5910465> [retrieved on 20110707]
- BARACHANT A ET AL: "Multiclass Brain Computer Interface classification by Riemannian geometry", IEEE TRANSACTIONS ON BIOMEDICAL ENGINEERING, vol. 59, no. 4, April 2012 (2012-04-01), pages 920 - 928, XP011490017, ISSN: 0018-9294, DOI: 10.1109/TBME.2011.2172210
- LI Y ET AL: "Electroencephalogram signals classification for sleep-state decision - a Riemannian geometry approach", IET SIGNAL PROCESSING, vol. 6, no. 4, 12 June 2012 (2012-06-12), pages 288 - 299, XP006041571, ISSN: 1751-9675, DOI: 10.1049/IET-SPR.2011.0234

**Description**

L'invention se rapporte au domaine technique de la commande neuronale directe, ou interface cerveau-machine, connu également sous l'acronyme « BCI », de l'anglais « brain-computer interface » (littéralement, « interface cerveau-ordinateur »).

Les interfaces cerveau-machine permettent d'établir une communication entre un utilisateur et une machine à travers des signaux neuronaux issus de l'activité cérébrale d'un sujet sans recourir à la voie musculaire, ce qui constitue un réel espoir pour les personnes souffrant de graves paralysies.

Les interfaces cerveau machine non-intrusives utilisent, le plus souvent, l'électroencéphalographie (EEG) comme méthode d'acquisition de l'activité cérébrale. On place ainsi un certain nombre d'électrodes à la surface du crâne dans le but d'y mesurer une activité électrique reflétant l'activité cérébrale du sujet. D'autres techniques plus intrusives exploitent des signaux électrocorticographiques (ECoG), prélevés à la surface du cortex, voire des signaux prélevés d'électrodes profondes.

Dans une interface cerveau machine, on associe une ou plusieurs tâches mentales (action imaginées par le sujet) à une ou plusieurs actions réalisées par un effecteur. Par exemple, l'imagination du mouvement de la main droite peut être associée au déplacement vers la droite d'un curseur. Le problème fondamental qui se pose et donc celui de détecter la réalisation de ces tâches mentales parmi l'ensemble des activités cérébrales que l'on mesure ; cette détection déclenche ensuite l'actionnement de l'effecteur, qui réalise l'action voulue.

La détection des tâches mentales (ou actions imaginées) comprend généralement plusieurs étapes. Premièrement, l'on applique aux signaux neuronaux « bruts » un filtrage destiné à rehausser les caractéristiques fréquentielles, temporelles et spatiales propres à chaque tâche mentale. Puis, des « caractéristiques » sont extraites des signaux ainsi filtrés. Enfin un algorithme de classification est appliqué à ces caractéristiques. Autrement dit, chaque ensemble de caractéristiques, correspondant aux signaux neuronaux acquis sur une période déterminée (on parle de « epoch », ou « époque ») est associé à une « classe » qui correspond à une tâche mentale déterminée - ou à sa négation. Ainsi, pour commander le déplacement d'un curseur le long d'une ligne il faut :

- une première tâche mentale (par exemple : un mouvement imaginaire de la main droite) associée à une première direction de déplacement du curseur (par exemple vers la droite) ;
- une deuxième tâche mentale (par exemple : un mouvement imaginaire de la main gauche) associée à une deuxième direction de déplacement du curseur par exemple vers la gauche) ; et
- trois classes, correspondant à la première et à la deuxième tâche mentale, ainsi qu'à toute autre forme d'activité cérébrale non reconductible à l'une de ces deux tâches.

L'étape de classification s'appuie sur les caractéristiques extraites d'un jeu de données d'entrainement appartenant à une classe déterminée et connue. Soit $\{(x_1, y_1), \ldots, (x_n, y_n)\}$ un jeu de données d'entrainement avec $x_i$ l'ensemble des caractéristiques du signal à l'instant i et $y_i$ la classe (et donc la tâche mentale) qui lui est associée. Entraîner un « classificateur », c'est trouver la fonction $h : X \rightarrow Y$ qui mappe les caractéristiques à leurs classes. Classifier un point x revient alors à calculer sa classe y = h(x). La qualité de la fonction h(.) dépend fortement de la qualité des caractéristiques qui servent à la calculer. Ainsi si les caractéristiques sont fortement bruitées, le taux d'erreur sera élevé. C'est pour cette raison que les caractéristiques ne sont généralement pas calculées sur les signaux bruts, mais sont extraites à partir de signaux préalablement filtrés.

L'article de F. Lotte, M. Congedo, A. Lécuyer, F. Lamarche, et B. Arnaldi, « A review of classification algorithms for EEG-based brain-computer interfaces » Journal of Neural Engineering, vol. 4, 2007, fournit une revue des nombreuses méthodes de classification utilisées en BCI.

La méthode la plus couramment utilisée comporte :

- Une étape de prétraitement constituée de filtrage fréquentiel et de filtrage spatial par une méthode connue sous le nom anglais « Common Spatial Pattern (CSP) », que l'on peut traduire par « caractéristiques spatiales communes ».
- Une étape d'extraction de caractéristiques consistant à calculer le logarithme de la variance des signaux filtrés.
- Une étape de classification par analyse discriminante linéaire de Fisher (Fisher LDA) sur les caractéristiques précédemment calculées.

Cette méthode a généralement des bonnes performances, est simple d'utilisation et peu coûteuse en calcul. Par contre elle est peu robuste (un point aberrant dans les données d'entrainement peut facilement corrompre les résultats), elle nécessite un nombre relativement important de données d'entrainement et elle exploite des caractéristiques de haut niveau, nécessitant le passage par plusieurs prétraitements, notamment la CSP qui est une technique supervisée, ce qui peut être gênant pour certaines applications, telles que les « chaînes non supervisées ». Globalement, elle s'avère peu efficace dès lors qu'il y a plus de deux classes.

L'information spatiale est importante pour faciliter la classification des signaux neuronaux. En effet, l'effectuation de tâches mentales différentes active différentes régions du cerveau, ou les mêmes régions mais d'une manière différente. Pour préserver au maximum cette information spatiale on utilise, dans la plupart des cas, un grand nombre d'électrodes (jusqu'à une centaine). Cette approche présente plusieurs inconvénients : une gêne pour l'utilisateur, un temps de préparation long, un coût calculatoire élevé. En outre, certains types de traitements montrent des limitations quand le nombre d'électrodes augmente (par exemple, certains classificateurs sont sujet au sur-apprentissage).

Ainsi, des techniques ont été développées pour sélectionner de manière optimale un nombre restreint d'électrodes.

On distingue typiquement entre « sélection montante », dans laquelle on construit progressivement un (petit) ensemble optimal d'électrodes, « sélection descendante » dans laquelle on sélectionne un sous-ensemble optimal à partir d'un ensemble de plus grandes dimensions, et « sélection descendante-montante » qui combine les deux approches précédentes. On distingue également entre sélection « patient-spécifique », dans laquelle on sélectionne un ensemble optimale d'électrodes pour un utilisateur déterminé, et sélection « application-spécifique » dans laquelle on cherche à déterminer un ensemble d'électrodes commun à tous les utilisateurs et dépendant de l'application spécifique, c'est-à-dire des tâches mentales à détecter.

Par exemple, l'article de A. Barachant, T. Aksenova, et S. Bonnet, « Filtrage spatial robuste à partir d'un sous-ensemble optimal d'électrodes en BCI EEG » GRETSI 2009, 8 - 11 septembre 2009, décrit une méthode de sélection d'électrodes ascendante et patient-spécifique basée sur un critère de corrélation multiple de la log-variance des signaux après filtrage fréquentiel. Les principaux inconvénients de cette méthode résident dans la sous-exploitation de l'information spatiale et la relative complexité de mise en oeuvre de l'algorithme.

L'invention vise à surmonter les inconvénients précités de l'art antérieur. En particulier l'invention vise :

- d'une part à procurer une méthode de classification de signaux neuronaux - et particulièrement de classification multiclasse (plus de deux classes) - qui soit à la fois robuste, simple à mettre en oeuvre, nécessitant de peu de données d'entrainement et de paramètres à régler ;
- d'autre part à procurer une méthode de sélection des électrodes d'une interface neuronale directe qui soit à la fois robuste, simple et flexible.

La méthode de classification et la méthode de sélection d'électrodes de l'invention peuvent être mises en oeuvre indépendamment l'une de l'autre : l'un des avantages de la méthode de sélection est justement sa flexibilité, c'est-à-dire sa possibilité d'être combinée avec différents algorithmes de classification. Les meilleurs résultats proviendront de leur utilisation combinée. Mais la sélection d'électrodes basée sur un critère de distance riemannienne améliore aussi très fortement les performances des méthodes de classification traditionnelles, mettant en oeuvre un filtrage CSP, car elle confère de la robustesse à ce filtrage.

Ces méthodes sont particulièrement adaptées à la BCI utilisant des signaux EEG, mais il ne s'agit pas là d'une limitation fondamentale.

La méthode de classification et la méthode de sélection d'électrodes de l'invention partagent une même approche, qui se base sur l'utilisation, en tant que « caractéristiques » descriptives des signaux neuronaux, de leurs matrices de covariance. Il s'agit là de caractéristiques relativement « proches » des signaux bruts, ce qui assure la robustesse de la méthode et permet une utilisation quasi-optimale de l'information disponible. Or, les matrices de covariance sont des matrices qui peuvent avoir des dimensions relativement importantes ; il pourrait donc sembler difficile de les utiliser telles quelles dans un classificateur ou dans un procédé de sélection d'électrodes. L'invention surmonte cette difficulté grâce au fait que les matrices de covariance sont symétriques et définies positives ; elles peuvent donc être considérées comme des points d'une variété riemannienne. Le classificateur et l'algorithme de sélection des électrodes se basent alors sur la géométrie riemannienne d'une telle variété. Le recours à la géométrie riemannienne s'avère d'ailleurs particulièrement avantageux pour une bonne classification des matrices de covariance.

Le document US2008/0063285 décrit une méthode de reconnaissance de figures humaines dans des images en mouvement basée sur la géométrie riemannienne des matrices de covariance. Il s'agit là d'un domaine technique tout à fait différent de la BCI.

Des procédés de classification de signaux sont décrits dans les documents suivants : la demande de brevet US 2008/063264 A1, l'article de W. Gersch et al. : « A Kullback Leibler-nearest neighbor rule classification of EEGs : The EEG population screening problem, an anesthésia level EEG classification application », Computers and Biomedical Research, vol. 13, n°. 3, juin 1980, pages 283-296, l'article de B. Kamousi et al. : « Classification of motor imagery by means of cortical current density estimation and Von Neumann entropy », Journal of Neural Engineering, vol.4, n°.2, juin 2007, pages 17-25, et l'article de Yili Li et al. : « EEG signal classification based on a Riemannian distance measure », Proceedings of 2009 IEEE Toronto International Conférence Science and Technology for Humanity (TIC-STH 2009), 26-27 September 2009, Ontario, Canada, pages 268-273;

L'article de F. Barabaresco et al. : "Espace Riemannien symétrique et géométrie des espaces de matrices de covariance: équations de diffusion et calculs de médianes", Actes du Colloque GRETSI 2009, 8-11 septembre 2009, Dijon, France, décrit l'utilisation des matrices de covariances en traitement du signal dans le cadre d'un espace Riemannien muni d'une métrique Riemannienne.

L'article de C. Sannelli et al. : « On optimal channel configuration for SMR-based Brain-Computer interfaces », Brain Topography, vol. 23, n° 2, 17 février 2010, pages 186-193, décrit un procédé de sélection d'électrodes d'acquisition de signaux neuronaux pour commande neuronale directe.

Un premier objet de l'invention est un procédé de classification de signaux neuronaux comportant les étapes suivantes :

    a. Acquérir, au moyen d'une pluralité d'électrodes et sur une période déterminée, une pluralité d'échantillons de signaux neuronaux;
    b. Estimer une matrice de covariance desdits signaux neuronaux ;
    c. Classifier lesdits signaux neuronaux, la classification étant réalisée :

        ○ soit dans la variété riemannienne des matrices symétriques définies positives de dimensions égales à celles de ladite matrice de covariance,
        ○ soit dans un espace tangent de ladite variété riemannienne.

Selon un mode de réalisation préféré, la classification peut être réalisée en utilisant un critère basé :

    ○ Soit sur une distance riemannienne définie sur la variété riemannienne des matrices symétriques définies positives de dimensions égales à celles de ladite matrice de covariance ;
    ○ Soit sur une distance euclidienne définie sur un espace tangent de ladite variété riemannienne.

Selon des modes de réalisation particuliers de ce procédé :

- Ledit critère de classification peut être un critère de moindre distance riemannienne entre ladite matrice de covariance des signaux à classifier et des matrices symétriques définies positives représentant des centres des classes. En particulier, chacune desdites matrices symétriques définies positives représentant le centre d'une classe peut correspondre à la moyenne géométrique ou à la médiane, dans ladite variété riemannienne, de matrices de covariance de données d'entrainement associés à ladite classe.
- En variante, ledit critère de classification peut être un critère de k plus proches voisins, dans ladite variété riemannienne, entre ladite matrice de covariance des signaux à classifier et des matrices de covariance de données d'entrainement associées à des classes.
- Le procédé peut comporter, avant ladite étape c. de classification : la détermination d'un espace tangent de ladite variété riemannienne par rapport à une matrice symétrique positive correspondant à une moyenne géométrique, ou à une médiane, dans ladite variété riemannienne, d'un ensemble de matrices de covariance de données d'entrainement associées à des classes ; la projection de ladite matrice de covariance à classifier, ainsi que desdites matrices de covariance de données d'entrainement, dans ledit espace tangent ; l'application d'un filtrage dimensionnel dans ledit espace tangent ; et la rétroprojection des données filtrées dans ladite variété riemannienne pour y effectuer ladite étape c. de classification.
- En variante, ladite étape c. de classification peut être mise en oeuvre dans un espace tangent de ladite variété riemannienne, déterminé par rapport à une matrice symétrique positive correspondant à une moyenne géométrique, ou à une médiane, dans ladite variété riemannienne, d'un ensemble de matrices de covariance de données d'entrainement associés à des classes. En particulier, ladite étape c. de classification peut être mise en oeuvre par analyse discriminante linéaire de Fisher dans ledit espace tangent.
- Plus particulièrement, un tel procédé peut comporter : avant ladite étape c. de classification, la détermination d'un espace tangent de ladite variété riemannienne par rapport à une matrice symétrique positive correspondant à une moyenne géométrique, ou à une médiane, dans ladite variété riemannienne, d'un ensemble de matrices de covariance de données d'entrainement associés à des classes ; la projection de ladite matrice de covariance à classifier, ainsi que desdites matrices de covariance des données d'entrainement, dans ledit espace tangent ; l'application d'un filtrage dimensionnel dans ledit espace tangent ; et ensuite la mise en oeuvre de ladite étape c. de classification sur les données filtrées dans ledit espace tangent.
- Le procédé peut comporter une étape de prétraitement desdits signaux neuronaux par filtrage fréquentiel et centrage.
- Lesdits signaux neuronaux peuvent être des signaux électroencéphalographiques.

Un deuxième objet de l'invention est un procédé de commande neuronale directe comportant : une étape de classification de signaux neuronaux tel que décrit ci-dessus ; et une étape de génération d'un signal de commande d'un appareil en fonction du résultat de ladite étape de classification.

Un troisième objet de l'invention est un système comportant : un ensemble d'électrodes d'acquisition de signaux neuronaux ; et un dispositif de traitement de données adaptés pour recevoir lesdits signaux et les utiliser pour mettre en oeuvre un procédé de classification et/ou de commande neuronale directe tel que décrit ci-dessus. Le dispositif de traitement des donnés peut être un ordinateur - ou un processeur de traitement des signaux - programmé de manière opportune, c'est-à-dire équipé d'une mémoire stockant un programme (une liste d'instructions exécutables) pour la mise en oeuvre du procédé.

Un quatrième objet de l'invention est un procédé de sélection d'électrodes d'acquisition de signaux neuronaux pour commande neuronale directe, consistant à choisir un sous-ensemble d'électrodes ou d'emplacements pour électrodes parmi un ensemble plus vaste, de manière à maximiser la somme des distances riemanniennes entre des matrices de covariance représentatives de classes différents de signaux issus desdites électrodes.

Un tel procédé peut comporter en particulier les étapes consistant à :

a. Appliquer à un sujet un ensemble initial d'électrodes d'acquisition de signaux neuronaux ;
b. Acquérir, au moyen desdites électrodes et sur une pluralité de périodes déterminées, une pluralité d'échantillons de signaux neuronaux, les signaux correspondant à chaque période étant associés à une parmi une pluralité de classes ;
c. Estimer une matrice de covariance des signaux neuronaux issus de chaque période, chacune desdites matrices de covariance étant considérée comme un point d'une variété riemannienne ;
d. Pour chacune desdites classes, calculer une matrice symétrique définie positive représentant un centre de classe ; et
e. Sélectionner un sous-ensemble desdites électrodes de manière à maximiser la somme des distances riemanniennes entre lesdits centres des classes.

Il s'agit là d'une sélection descendante.
Selon différents modes de réalisation particuliers de ce procédé :

- Ladite étape d. peut consister à calculer la moyenne géométrique, ou la médiane, dans ladite variété riemannienne, des matrices de covariance des signaux associés à chaque classe.
- Ladite étape e. peut être mise en oeuvre de manière itérative et comporter, à chaque itération, l'élimination d'une électrode choisie de manière à minimiser la diminution de distance riemannienne entre les matrices de covariance moyennes ou médianes desdites classes qui en résulte.

En variante (sélection ascendante) le procédé de sélection peut comporter les étapes consistant à :

a. Déterminer un ensemble d'emplacements pour l'application d'électrodes d'acquisition de signaux neuronaux sur un sujet ;
b. Introduire progressivement des électrodes de manière à incrémenter de manière maximale ladite somme des distances riemanniennes entre des matrices de covariance représentatives de classes différentes de signaux issus desdites électrodes. Autrement dit, à chaque incrément, on ajoute l'électrode qui maximise l'augmentation de la distance riemannienne entre lesdites matrices de covariance

Que la sélection soit ascendante ou descendante :

- Le procédé peut conduire à la détermination d'un sous-ensemble d'électrodes comportant un nombre prédéterminé d'éléments.
- Chaque classe de signaux neuronaux peut correspondre à une action imaginée par le sujet.
- Chacune desdites électrodes peut être adaptée pour acquérir des signaux neuronaux générés par une région déterminée du cerveau du sujet.

Un cinquième objet de l'invention est un procédé de sélection d'électrodes d'acquisition de signaux neuronaux pour commande neuronale directe, comportant : la mise en oeuvre d'un procédé de sélection (ascendante ou descendante) tel que décrit ci-dessus pour une pluralité de sujets, avec un même ensemble initial d'électrodes ou d'emplacement pour électrodes pour tous les sujets, de manière à déterminer un sous-ensemble d'électrodes pour chaque sujet ; et la sélection des électrodes dudit ensemble initial qui appartiennent au plus grand nombre desdits sous-ensembles.

Un sixième objet de l'invention est un procédé de commande neuronale directe tel que décrit plus haut, comportant une opération préalable de sélection des électrodes d'acquisition de signaux neuronaux selon un procédé tel que décrit ci-dessus.

D'autres caractéristiques, détails et avantages de l'invention ressortiront à la lecture de la description faite en référence aux dessins annexés donnés à titre d'exemple et qui montrent, respectivement :

- La figure 1, une illustration des notions de variété riemannienne, géodésique, distance riemannienne et espace tangent ;
- La figure 2, le découpage des signaux neuronaux mis en oeuvre par les procédés de classification et de sélection d'électrodes de l'invention ;
- La figure 3, un ordinogramme illustrant différentes variantes du procédé de classification de l'invention ;
- La figure 4, un graphique illustrant un procédé de classification selon un premier mode de réalisation de l'invention, basé sur la mesure de la distance riemannienne par rapport à la moyenne ;
- La figure 5, un graphique illustrant un procédé de classification selon un deuxième mode de réalisation de l'invention, basé sur le critère des k plus proches voisins (au sens riemannien) ;
- La figure 6, une illustration de différents procédés de classification selon des modes de réalisation respectifs de l'invention, comportant une projection des données de classification dans un espace tangent à la variété riemannienne des matrices de covariance ;
- La figure 7, la géométrie d'implantation de 16 électrodes d'EEG pour BCI ;
- Les figures 8 et 9, l'application d'un procédé de sélection d'électrodes « patient-spécifique » à la géométrie d'implantation de la figure 7 ;
- La figure 10, les résultats de ce même procédé appliqué à huit utilisateurs différents ; et
- La figure 11, l'application d'un procédé de sélection d'électrodes « application-spécifique » aux utilisateurs de la figure 1.

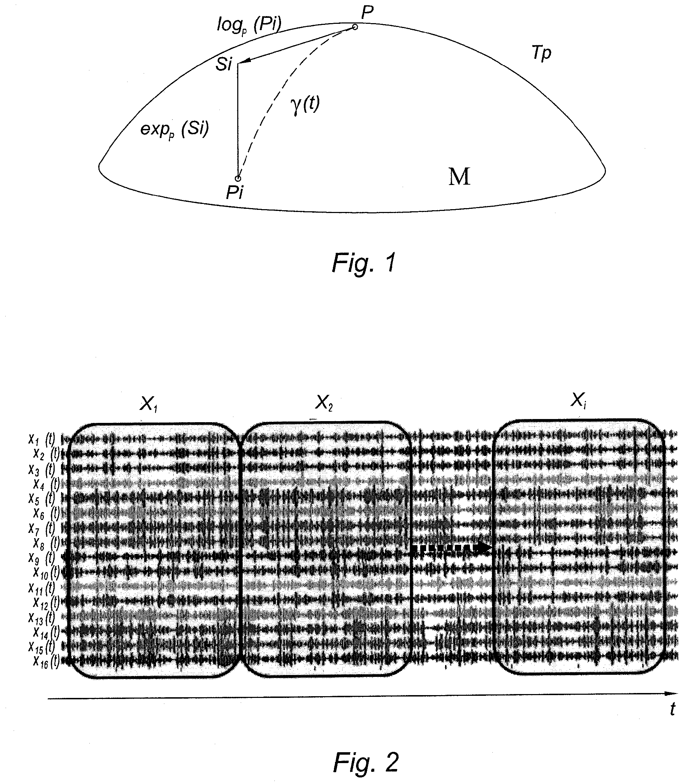

Il est opportun, tout d'abord, de définir quelques notions fondamentales de géométrie riemannienne à l'aide de la figure 1.

Une variété riemannienne est un espace topologique localement homéomorphe à un espace euclidien ( $\Re^n$ ), différentiable et sur lequel est défini un produit scalaire suffisamment régulier. Le produit scalaire permet à son tour de définir une géométrie (« géométrie riemannienne ») sur la variété riemannienne. Cette géométrie ne coïncide pas avec la géométrie euclidienne « usuelle » ; pour cette raison, sur la figure 1, la variété riemannienne M a été représentée (de manière symbolique) par une surface courbe.

Une matrice symétrique définie positive (SDP) est une matrice carrée symétrique par rapport à sa diagonale ($a_{ij}=a_{ji}$) et ayant des valeurs propres strictement positive. Une matrice SDP de dimensions nxn a n(n+1)/2 éléments indépendants ; elle peut donc être représentée dans un espace à n(n+1)/2 dimensions. Il est possible de démontrer que cet espace peut être muni de la structure d'une variété riemannienne. Dans la suite on considérera uniquement la variété M des matrices SDP de dimensions nxn, n étant tout naturel supérieur à 1.

La distance riemannienne entre deux points $P_1$, $P_2$ de la variété riemannienne M peut être définie par :

$$\delta_R(\mathbf{P}_1, \mathbf{P}_2) = \left\| \mathrm{Log}\left(\mathbf{P}_1^{-1}\mathbf{P}_2\right) \right\|_F = \left[\sum_{i=1}^{n} \log^2 \lambda_i\right]^{1/2}$$

Où $\|.\|_F$ est la norme de Frobenius et $\lambda_i$ sont les valeurs propres de $P_1^{-1}P_2$ (réelles est strictement positives). Le logarithme d'une matrice SDP « P » est donné par : Log (P) = Vlog(D)V$^{-1}$ où V est la matrice des vecteurs propres de P, D la matrice diagonale de ses valeurs propres et log(D) représente la matrice diagonales des logarithmes des valeurs propres de P.

Cette définition de la distance riemannienne n'est pas univoque. D'autres métriques équivalentes à $\delta_R$ peuvent être définies sur la variété M. Par exemple, une distance alternative pouvant être utilisée dans la mise en oeuvre de l'invention est définie par : $\|Log(P_1)-Log(P_2)\|_F$.

Une géodésique est le plus court chemin, sur la variété M, entre deux points $P_1$ et $P_2$. Elle est définie sous forme paramétrique, avec paramètre te[0,1] par :

$$\gamma(t) = \mathbf{P}_1^{1/2}\left(\mathbf{P}_1^{-1/2}\mathbf{P}_2\mathbf{P}_1^{-1/2}\right)^t \mathbf{P}_1^{1/2}$$

En correspondance de chaque point P de la variété riemannienne M il est possible de définir - de manière univoque - l'espace tangent T$_p$, qui est un espace euclidien (représenté symboliquement par un plan sur la figure 1). Plus précisément, donné un point P de la variété M, il est possible de définir pour chaque autre point P$_i$ de ladite variété le vecteur tangent S$_i$ = γ̇(0), où γ est la géodésique entre P et P$_i$ et le point représente l'opération de dérivation.

La variété M est localement (autour du point P) homéomorphe à l'espace tangent T$_p$ constitué par l'ensemble des vecteurs tangent S$_i$. A chaque point P$_i$ de la variété riemannienne M il est donc possible d'associer un point S$_i$ de l'espace tangent T$_p$ (c'est-à-dire un vecteur tangent) par une opération de projection généralement connue sous le nom anglais de « log map », définie par :

$$\text{Log}_{\mathbf{P}}(\mathbf{P}_i) = \mathbf{P}^{1/2} \text{Log}\left(\mathbf{P}^{-1/2}\mathbf{P}_i\mathbf{P}^{-1/2}\right)\mathbf{P}^{1/2}$$

Inversement, le passage de l'espace tangent à la variété M se fait par une opération de « exp map » :

$$\text{Exp}_{\mathbf{P}}(\mathbf{S}_i) = \mathbf{P}^{1/2} \text{Exp}\left(\mathbf{P}^{-1/2}\mathbf{S}_i\mathbf{P}^{-1/2}\right)\mathbf{P}^{1/2}$$

La puissance t d'une matrice SDP « P » est définie par $P^t = VD^tV^{-1}$ où V est la matrice des vecteurs propres de P, D la matrice diagonale de ses valeurs propres et D$^t$ la matrice diagonale des valeurs propres de P élevés à la puissance t.

Les éléments de l'espace tangent, au point P, de la variété riemannienne des matrices SDP de dimensions nxn sont des matrices symétriques, mais en générales non définies positives, des mêmes dimensions.

Une autre notion utile pour la suite est celle de moyenne géométrique au sens riemannien, de m matrices SDP appartenant à la variété M :

$$\mathfrak{G}\left(\mathbf{P}_1, \ldots, \mathbf{P}_m\right) = \underset{\mathbf{P}\in P(n)}{\text{argmin}} \sum_{i=1}^{m} \delta_R^2\left(\mathbf{P}, \mathbf{P}_i\right)$$

où P(n) représente l'ensemble des matrices SDP nxn.

Il n'existe pas de formule analytique permettant de calculer cette moyenne ; il est donc nécessaire d'avoir recours à des algorithmes itératifs.

Le plus simple de ces algorithmes consiste à :

1. Déterminer une première estimation P$^0_\Omega$ de la moyenne géométrique afin d'initialiser l'algorithme ; par exemple, on peut prendre la moyenne arithmétique des matrices.

2. Projeter les matrices P$_1$...P$_m$ dans l'espace tangent au point P$^0_\Omega$ (on remarquera qu'on utilise les termes « point » et « matrice » de manière interchangeable, selon qu'on veuille mettre en évidence les aspects géométriques ou algébriques).

3. Calculer la moyenne arithmétique S des projections des matrices, puis revenir à la variété riemannienne par application d'une « exp map ». On trouve une nouvelle estimation P$^1_\Omega$ de la moyenne (plus généralement, à l'itération i, on indiquera cette estimation par P$^i_\Omega$).

4. On itère les étapes 2 et 3, prenant à chaque fois l'espace tangent au point P$^{i-1}_\Omega$ déterminé à l'itération précédente. Le processus s'arrête lorsque la norme de Frobenius de la matrice S (moyenne géométrique dans l'espace tangent) calculée à l'étape 3 est inférieure à une valeur de seuil.

Il existe aussi des algorithmes permettant de calculer des estimateurs robustes de la moyenne. Pour plus de précisions concernant la géométrie riemannienne des matrices SDP il est possible de se rapporter à l'article de M. Moakher « A differential géométrie approach to the géométrie mean of symmetric Positive-Definite matrices » SIAM J. Matrix Anal. Appl., 26, 2005.

Comme cela a été expliqué plus haut, l'invention part de l'hypothèse que les matrices de covariance des signaux neuronaux - et en particulier des signaux EEG - constituent des bons indicateurs de l'activité cérébrale d'un sujet et peuvent être utilisées pour la réalisation d'interfaces neuronales directes, à la condition de considérer ces matrices comme des points d'une variété riemannienne, ce qui permet d'en préserver la structure et les relations.

La figure 2 explicite la notion de « matrice de covariance des signaux neuronaux ». Un sujet, ou patient, ou utilisateur (ces termes seront utilisés ici de manière interchangeable) est équipé de N électrodes pour EEG ou ECoG. Chaque électrode « i » fournit un signal $x_i(t)$ en fonction du temps. Ce signal est échantillonné, pour passer à un temps discret : $x_{ij}=x_i(t_j)$, puis numérisé. On obtient ainsi une représentation matricielle de l'ensemble des signaux neuronaux. Le temps global d'acquisition est découpé en périodes, connues dans la technique sous le nom anglais de « epochs ». A chaque période est donc associée une matrice $X_1$, $X_2$ .... $X_l$ représentative des signaux acquis au cours de ladite période. En pratique, ces matrices correspondent généralement à des signaux filtrés en fréquence, pour améliorer le rapport signal sur bruit et isoler une région spectrale d'intérêt, et centrés par soustraction de la moyenne, calculée ligne par ligne (en réalité, le « centrage » est une conséquence automatique du filtrage, dès lors que la composante continue du signal est éliminée). La matrice de covariance $P_i$ de la i_ème période peut être estimée par :

$$P_i = \frac{1}{M-1} X_i X_i^T$$

Où *M* est le nombre d'échantillons de la période pour chaque électrode (à ne pas confondre avec M, qui indique la variété riemannienne). D'autres estimateurs plus robustes, connus par eux-mêmes, peuvent également être utilisés.

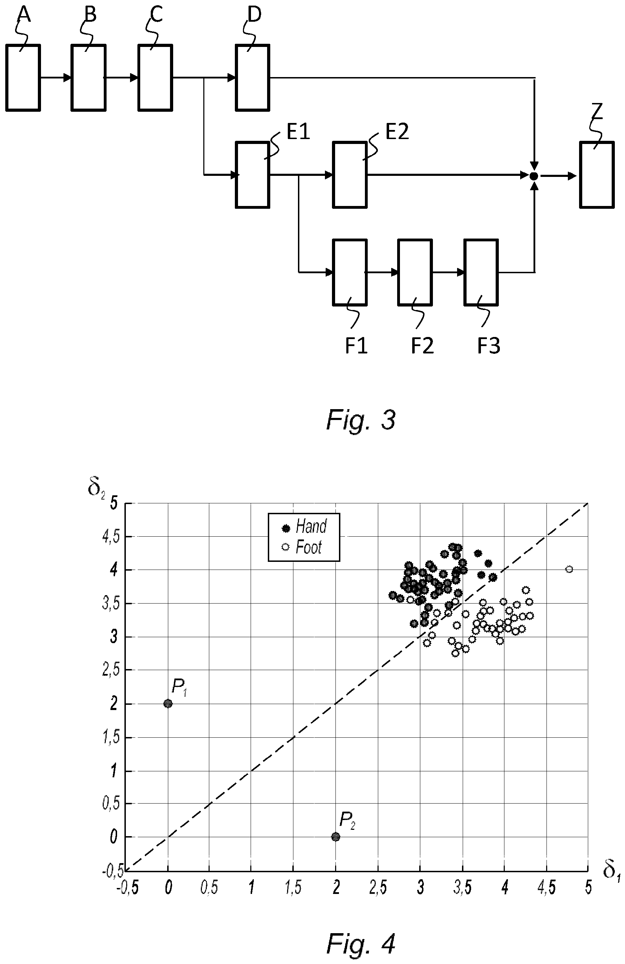

La figure 3 montre un ordinogramme général d'un procédé selon l'invention.

L'étape A consiste à acquérir les signaux neuronaux à l'aide d'un ensemble d'électrodes (on verra plus loin comment déterminer de manière optimale cet ensemble).

L'étape B comprend des opérations conventionnelles de prétraitement : échantillonnage, conversion au format numérique, filtrage, centrage, normalisation...

L'étape C consiste en l'estimation des matrices de covariance associées aux différentes périodes, considérées comme des points d'une variété riemannienne M.

L'étape D consiste à classifier les matrices de covariance dans ladite variété riemannienne. Le classificateur est généré à partir de données d'entrainement, c'est-à-dire des matrices de covariance de périodes appartenant à des classes connues (des périodes pendant lesquelles le sujet a effectué une tâche mentale prédéfinie).

Enfin, l'étape Z consiste à générer des signaux de commande d'un effecteur à partir des résultats de la classification. La classification des signaux neuronaux peut servir aussi à d'autres fins que la BCI (recherche, diagnostic...), auquel cas on pourra omettre ladite étape Z.

Les étapes E1 et E2 peuvent être mises en oeuvre en alternative à l'étape D. Elles consistent à :

- E1 : projeter les matrices de covariance dans un espace tangent (généralement, le point correspondant à la moyenne géométrique au sens riemannien des données d'entrainement) ;
- E2 : effectuer la classification dans ledit espace tangent.

Cette approche alternative est intéressante en ce qu'elle permet d'appliquer des classificateurs « sophistiqués » qui ne peuvent pas être utilisés directement dans une variété riemannienne, tels que l'analyse discriminante linéaire de Fisher (LDA).

Une alternative à l'étape E2 est constituée par F1, F2, F3 :

- F1 consiste à appliquer une opération de « filtrage dimensionnel » dans l'espace tangent. Le « filtrage dimensionnel » se différencie de la réduction dimensionnelle en ce que la dimension des données est maintenue constante par remplissage avec des zéros.
- F2 consiste à projeter les données filtrées dimensionnellement dans la variété riemannienne d'origine (« exp map »).
- Enfin, F3 consiste à classifier les données filtrées dimensionnellement dans la variété riemannienne.

La figure 4 illustre une première méthode de classification de signaux neuronaux dans une variété riemannienne : la méthode dite de la « distance à la moyenne » (ou « à la médiane »). Le principe consiste à définir, sur la variété riemannienne, des points (matrices) dits « centres des classes ». Il y a un - et un seul - de ces points pour chaque classe. Sur la figure 4 il y a deux de ces points, $P_1$ et $P_2$ correspondant à deux tâches mentales distinctes : un mouvement imaginé d'une main et un mouvement imaginé d'un pied. L'on calcule la distance riemannienne $\delta_1(P_i)$ - respectivement $\delta_2(P_i)$ - entre chaque matrice covariance d'une période d'activité cérébrale à classifier et le point $P_1$ - respectivement $P_2$. Si $\delta_1(P_i) < \delta_2(P_i)$, alors $P_i$ est associée à la classe n°1 (mouvement de la main), sinon elle est associée à la classe n°2 (mouvement du pied). La figure 4 montre les représentations de $P_1$, $P_2$ et de plusieurs matrices de covariance à classifier dans un plan $\delta_1/\delta_2$. La classification « réelle » de ces matrices de covariance est connue : les points en gris foncé correspondent à des périodes pendant lesquelles le sujet a imaginé un mouvement de la main (ces points devraient donc être associés à la première classe) ; les points en gris clair correspondent à des périodes pendant lesquelles le sujet a imaginé un mouvement du pied (ces points devraient donc être associés à la deuxième classe). On peut vérifier que la classification est correcte pour la grande majorité des points.

Se pose le problème de déterminer les « centres des classes ». Une manière de procéder est la suivante. Plusieurs signaux d'entraînement sont acquis : il s'agit de matrices de covariance de périodes d'activité cérébrale dont la classification réelle est connue (mouvement imaginé de la main ou du pied). Les points qui représentent ces matrices forment des « nuages » dans la variété riemannienne M. Le point $P_1$ est défini comme la moyenne géométrique (au sens riemannien) des matrices de covariance des signaux d'entrainement correspondant à un mouvement imaginé de la main (donc des matrices d'entrainement appartenant à la première classe). Le point $P_1$ est défini comme la moyenne géométrique (au sens riemannien) des matrices de covariance des signaux d'entrainement correspondant à un mouvement imaginé de la main (donc des matrices d'entrainement appartenant à la première classe). En variante, le centre de classe $P_1$ peut correspondre à la médiane des matrices de covariance des signaux d'entrainement. La médiane dans la variété riemannienne est définie comme le point qui minimise la somme des distances riemanniennes (et non de leurs carrés). Elle peut être déterminée grâce à un algorithme itératif, comme la moyenne ; mais la convergence de cet algorithme est plus délicate.

Dans la méthode de la distance à la moyenne (médiane), les données d'entrainement servent uniquement à calculer les « centre de classes », après quoi elles peuvent être oubliées. Dans une méthode alternative, dite « des k plus proches voisins », ces données jouent au contraire un rôle central. Conformément à cette méthode, pour chaque « point » de la variété riemannienne correspondant à une matrice de covariance à classer, on détermine les k « points » correspondant aux matrices d'entrainement qui sont les plus proches au sens riemannien. En général, ces « k plus proches voisins » appartiendront à des classes différentes. Le point à classifier sera attribué à la classe à laquelle appartient la majorité de ses k plus proches voisins.

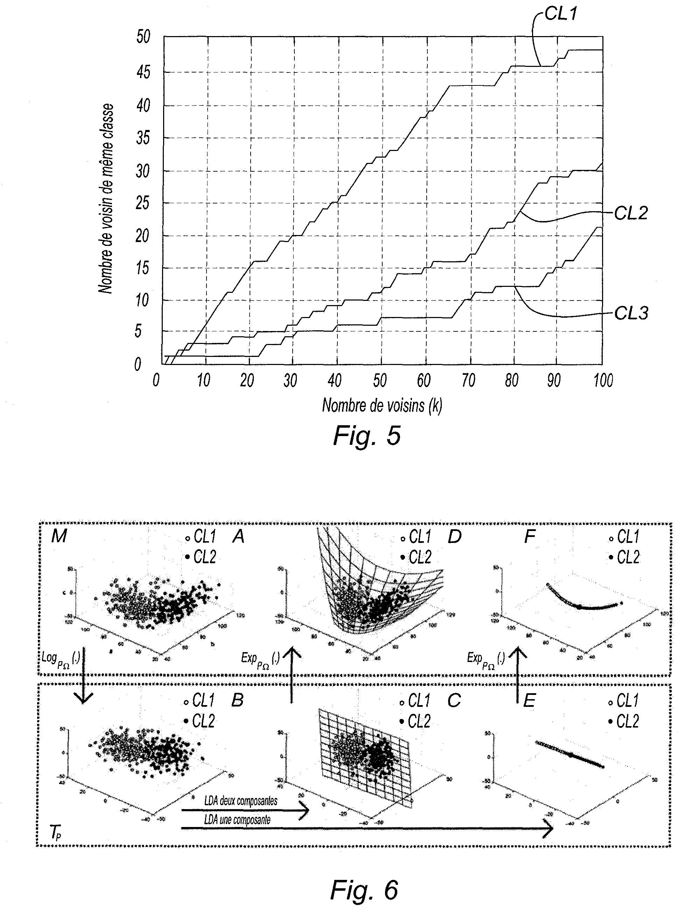

Dans l'exemple de la figure 5 on utilise 100 points d'entrainement associés à 3 classes (« main » : CL1, « pied » : CL2, « repos » : CL3). On étudie la classification d'une période d'activité cérébrale que l'on sait associée à un mouvement imaginé de la main. L'axe des abscisses représente le nombre k de plus proches voisins considérés ; l'axe des ordonnées représente le nombre de plus proches voisins par classe. On peut voir que déjà pour k=10 la période est attribuée de manière non ambigüe à la catégorie correcte.

Les exemples des figures 4 et 5 correspondent à la branche « D » de l'ordinogramme de la figure 3 (classification directement dans la variété riemannienne). On considère maintenant la branche « E1/E2 », c'est-à-dire la classification dans l'espace tangent.

On sait que la variété riemannienne M possède un espace tangent pour chacun de ses points. Se pose donc la question de déterminer l'espace tangent dans lequel projeter les données afin de les classifier. Pour minimiser la distorsion des données provoquée par la projection, il est préférable de choisir l'espace tangent relatif à la moyenne géométrique des données d'entrainement. Selon une variante, on peut choisir l'espace tangent relatif à la médiane des données d'entrainement.

A chaque « point » (matrice SDP) Pi de la variété riemannienne M correspond un « point » Si dans l'espace tangent considéré. Or, $S_i$ est une matrice seulement symétrique. Il y a donc redondance de l'information dans ses coefficients. On introduit l'opération :

$$\widetilde{S}_i = vect(S_i)$$

consistant à ne garder que les coefficients de la partie supérieure de la matrice et de les ranger sous forme de vecteurs, comme l'illustre l'exemple suivant avec une matrice 3x3

$$S_i = \begin{bmatrix} 1 & 2 & 3 \\ 2 & 4 & 5 \\ 3 & 5 & 6 \end{bmatrix} \xrightarrow{vect} \widetilde{S}_i = \begin{bmatrix} 1 \\ 2 \\ 3 \\ 4 \\ 5 \\ 6 \end{bmatrix}$$

$S_i$ étant une matrice de dimension nxn, alors $\widetilde{S}_i$ est un vecteur de n*(n+1)/2 éléments.

On créer donc un nouveau jeu de donnée sur lequel on entraînera un classificateur :

$$\left\{ \left( \widetilde{S}_1, Y_1 \right) \dots \left( \widetilde{S}_i, Y_i \right) \dots \left( \widetilde{S}_{180}, Y_{180} \right) \right\}$$

Ou $Y_i$ est la classe « réelle » du vecteur d'entrainement $\widetilde{S}_i$.

Ce classificateur peut être, par exemple, une analyse discriminante linéaire de Fisher.

Il y a cependant une difficulté. En effet, les vecteurs $\widetilde{S}_i$ sont de taille n*(n+1)/2 et lorsque le nombre d'électrodes devient important (de l'ordre de 20 ou plus), la taille de ces vecteurs se rapproche du nombre total de points dans la base de données (en général, 200 à 500 en BCI), voire dépasse ce nombre. Les algorithmes classiques type LDA ou régression le supportent mal et tendent au sur-apprentissage. Pour la LDA en particulier, la résolution est impossible si la taille des vecteurs dépasse le nombre de points du jeu de données. On utilise alors soit une réduction de dimension, soit des méthodes de régularisations. Ces méthodes font partie intégrante de l'état de l'art pour les techniques de classification. Une méthode simple de régularisation consiste augmenter artificiellement le nombre de données dans la base d'apprentissage, soit par ré-échantillonage desdites données, soit par introduction de bruit (chaque point est dupliqué et déplacé légèrement selon un bruit blanc gaussien).

La réduction dimensionnelle peut se faire, par exemple, par Analyse Discriminante Linéaire de Fisher, ou « Fischer LDA » (R. O. Duda, P. E. Hart, D.G. Stork. « Pattern Classification », chapitre 3.8.2, pages 117-124. J. Wiley & Sons Inc, 2e édition, 2000). Cette méthode projette les données sur des vecteurs hiérarchisés par importance, dont seul un nombre K (généralement petit par rapport à la dimension totale des données) est conservé ; la projection sur un seul vecteur peut être considérée comme une classification binaire (le signe de la projection donne directement la classe d'appartenance). A ce point, on peut effectuer la classification dans l'espace tangent, ou revenir dans la variété riemannienne. Pour ce faire, il faut revenir à une représentation des données ayant les dimensions d'origine ; mais la valeur des dimensions autres que les K retenues après Analyse Discriminante Linéaire de Fisher est posée égale à zéro. Comme il n'y a pas de réduction dimensionnelle proprement dite, on parle de « filtrage dimensionnel ».

La figure 6 montre :

A : Des données de deux classes différentes (points gris clair et gris foncé) dans une variété riemannienne à 3 dimensions, correspondant à des matrices SDP de dimensions 2x2 (bien entendu, il s'agit seulement d'un exemple à des fins de visualisation ; les matrices de covariance utilisées en BCI ont des dimensions beaucoup plus importantes, qui ne permettent pas une visualisation aisée). La classification peut se faire directement dans la variété riemannienne.

B : La projection de ces données dans un espace tangent. On perd la « courbure » de la variété riemannienne, car l'espace tangent est euclidien. La classification peut être effectuée dans cet espace tangent.

C : On applique une LDA de Fisher à deux composantes. Les données sont aplaties sur un plan (espace euclidien à deux dimensions), choisi de manière à optimiser la séparation des classes. On peut effectuer la classification sur cet espace euclidien de dimensions réduites.

D : A condition de considérer le plan obtenu au point C comme immergé dans l'espace tangent à trois dimensions, il est possible de revenir dans la variété riemannienne. Les données à classifier sont agencées sur une surface (en général : hypersurface) de la variété riemannienne.

E : A partir du point B on peut appliquer une LDA de Fisher à une composante. Les données sont concentrées sur une droite, correspondant à la direction de plus grande séparation des classes. La classification est particulièrement aisée, mais on a perdu de l'information. Cette perte d'information peut être acceptable lorsqu'on veut faire de la classification à deux classes ; en revanche, l'utilisation d'une seule composante est généralement insuffisante pour faire une classification multiclasses.

F : A partir du point E on revient dans la variété riemannienne. Les données apparaissent concentrées sur une ligne, plus précisément une géodésique (la géodésique de plus grande séparation entre les classes).

On considère maintenant un exemple spécifique d'interface cerveau-machine utilisant 16 électrodes d'EEG et trois tâches mentales : mouvement d'une main, d'un pied et repos. Les données d'entrainement du classificateur sont obtenues en demandant à l'utilisateur d'effectuer 20 fois chacune des tâches mentales pendant 3 secondes/tâche. Les signaux neuronaux sont échantillonnés à 512 Hz, filtrés par un filtre passe-bande entre 8 et 30 Hz, puis découpés en portions d'une seconde (il y en a donc 180), pour chacune desquelles on détermine une matrice de covariance $X_i$. Chacune de ces matrices de covariance - de dimensions 16x512 - est associée à un scalaire $Y_i$ qui indique la tâche mentale correspondante : 1 pour le mouvement de la main, 2 pour le mouvement du pied et 3 pour le repos.

Ces données sont utilisées avec plusieurs types de classificateurs :

- Une chaîne de traitement « conventionnelle » : CSP suivie par LDA multiclasse ;
- Une classification par distance à la moyenne dans la variété riemannienne des matrices $X_i$ ;
- Une classification par k plus proches voisins (k=20) dans la variété riemannienne des matrices $X_i$ ;

- Une classification par LDA dans l'espace tangent ;

et cela pour 9 utilisateurs U1 - U9.

Les résultats, exprimés en pourcentage de bonne classification, sont donnés par la matrice suivante. L'avant-dernière ligne donne les résultats moyens et la dernière ligne le gain par rapport à la méthode de référence. Il faut considérer qu'une classification au hasard donnerait un résultat égal à 33,33%.

| Utilisateur | Référenc e (CSP + LDA) | Distance à la moyenne | k plus proches voisins | LDA dans l'espace tangent |
|---|---|---|---|---|
| U1 | 34,9 | 55,0 | 51,0 | 49,7 |
| U2 | 48,3 | 61,6 | 57,6 | 51,7 |
| U3 | 44,7 | 44,1 | 36,2 | 40,8 |
| U4 | 73,4 | 76,0 | 73,4 | 67,1 |
| U5 | 50,0 | 58,4 | 57,1 | 50,7 |
| U6 | 38,5 | 67,1 | 62,1 | 62,8 |
| U7 | 31,5 | 37,0 | 39,5 | 30,9 |
| U8 | 55,5 | 58,7 | 55,5 | 44,6 |
| U9 | 65,4 | 57,7 | 64,7 | 64,2 |
| Moyenne | 49,1 | 57,3 | 55,2 | 51,4 |
| Gain (%) | | +8,2 | +6,1 | +2,3 |

Les méthodes de classification basées sur la géométrie riemannienne fournissent une augmentation des performances significative, sauf pour la LDA dans l'espace tangent qui, dans les conditions considérées ici est proche d'un sur-apprentissage (en effet, avec 16 électrodes, les vecteurs $\tilde{S}_i$ sont de dimension 136, proche du nombre de points d'entrainement, 180).

Les méthodes de classification considérées jusqu'ici sont de type « supervisé », c'est à dire utilisant des signaux d'apprentissage dont la classe est connue. L'invention s'applique également aux méthodes de classification non supervisées, bien que leur utilisation en BCI soit généralement plus délicate. A titre d'exemple on peut mentionner la généralisation riemannienne de la méthode des « k moyennes » (« k-means » en anglais), connue par elle-même. Un procédé de classification non supervisée de signaux neuronaux selon l'invention comporte les étapes suivantes :

a. On acquiert des signaux neuronaux sur I périodes temporelles et, après un prétraitement éventuel, on en détermine les matrices de covariance ;
b. On attribue, de manière aléatoire, chaque matrice de covariance - considérée comme un point d'une variété riemannienne - à une parmi k classes ;
c. On calcule le centre au sens riemannien (moyenne géométrique ou médiane) de chaque classe ;
d. On calcule la distance riemannienne entre chaque matrice et les k centres des classes ; puis on réattribue chaque matrice à la classe dont le centre est plus proche ;

Puis on répète de manière itérative les étapes c. et d. jusqu'à la convergence.

Lorsqu'un nouveau « point » (une nouvelle période d'acquisition) se présente, on peut recommencer l'algorithme sur l'ensemble de données augmenté. En variante, on peut fixer définitivement les centres des classes après une phase d'apprentissage (non supervisé), et utiliser simplement une classification par distance à la moyenne (médiane) pour les données acquises ultérieurement.

L'utilisation de la géométrie riemannienne dans les techniques de classification non supervisée est de permettre une bonne exploitation de l'information spatiale, alors même qu'un filtrage par CSP n'est pas disponible (car supervisé).

Jusqu'ici on a considéré l'ensemble d'électrodes (leur nombre, leur agencement sur le crâne de l'utilisateur) comme une donnée. En réalité, il s'agit là de paramètres à régler afin d'optimiser les performances de la BCI. La géométrie riemannienne des matrices de covariance des signaux neuronaux s'avère utile, outre que pour la classification proprement dite, pour permettre la détermination de la disposition des électrodes d'acquisition des signaux.

L'idée générale est de sélectionner un certain nombre d'électrodes, parmi un ensemble plus vaste, de manière à maximiser la distance riemannienne entre les centres des classes des données d'entrainement (ou plus généralement entre des matrices « représentant » les différentes classes). S'il y a plus de deux classes, on cherchera à maximiser une fonction de distances entre les centres des classes pris deux à deux (typiquement, on prendra leur somme :

$$Critère = \sum_{k=1}^{K-1} \sum_{j>k}^{K} \delta_R\left(P_k, P_j\right)$$

, où $\delta_R$ est la distance riemannienne, $P_k$, $P_j$ sont les centres des classes et la somme est étendue à toutes les paires de classes.

En effet, l'ajout d'une électrode augmente la dimension des matrices X, et donc de la variété riemannienne ; en général, cela se traduit par une augmentation de la distance entre les centres des classes. Si cette augmentation ne se produit pas, cela signifie que l'électrode ajoutée ne porte aucune information utile à la classification. Réciproquement, le fait d'enlever une électrode diminue, en général, la distance entre les centres de classe. Si cette diminution est importante, cela signifie que l'électrode enlevée portait beaucoup d'information utile (et donc qu'il aurait probablement dû être conservée). Si cette diminution est faible ou nulle, cela signifie que cette électrode ne portait que peu d'information utile (et donc que son élimination permet de simplifier le système sans dégradation notable de ses performances).

Cette approche convient particulièrement bien à la sélection descendante. On procède de la manière suivante :

On part d'un ensemble « complet », comprenant un nombre important N d'électrodes et on acquiert les signaux d'entrainement.

Puis on calcule les matrices de covariance, on détermine le centre des classes et on calcule le « critère » (somme des distances entre lesdits centres des classes).

Puis on considère tous les sous-ensembles de N-1 électrodes (obtenues en éliminant une électrode de l'ensemble complet). On calcule les nouvelles matrices de covariance et la nouvelle valeur du « critère » pour chacun de ces sous-ensembles. Le sous-ensemble présentant la valeur du « critère » la plus élevée sera retenue : il s'agit du sous-ensemble obtenu en éliminant de l'ensemble complet l'électrode qui apporte le moins d'information utile à la classification.

Et ainsi de suite : à chaque étape on teste tous les sous-ensembles obtenus en éliminant une électrode et on sélectionne le meilleur de ces sous-ensembles (celui qui optimise le critère défini par la distance entre les centres des classes). De cette manière on est certains d'éliminer, à chaque étape, l'électrode la moins « utile » parmi celles qui restaient.

Le processus de sélection s'arrête quand on reste avec un nombre prédéfini d'électrodes ; ou lorsque la valeur du « critère » descend au-dessous d'un seuil (par exemple : 80% de la valeur initiale).

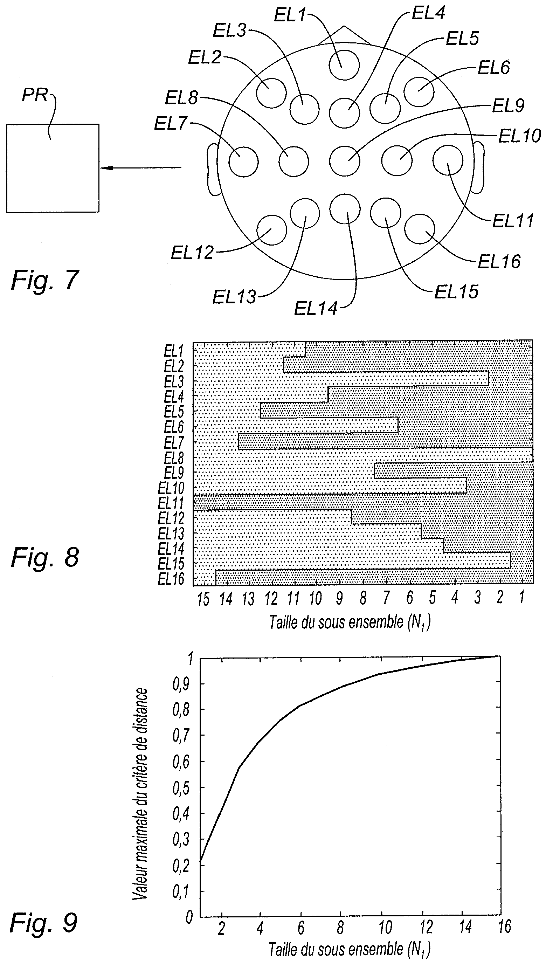

A titre d'exemple, la figure 7 montre un montage initial de 16 électrodes EL1 - EL16 que l'on souhaite réduire de manière optimale. Comme dans l'exemple étudié plus haut pour illustrer les différentes méthodes de classification des signaux neuronaux, l'on considère trois tâches mentales (mouvement d'une main, d'un pied et repos) et des données d'entrainement obtenues en demandant à l'utilisateur d'effectuer 20 fois chacune des tâches mentales pendant 3 secondes/tâche. Les signaux neuronaux sont échantillonnés à 512 Hz, filtrés par un filtre passe-bande entre 8 et 30 Hz, puis découpés en portions d'une seconde.

La figure 7 montre également, de manière schématique, un processeur PR programmé pour mettre en ouvre un procédé de classification et de BCI tel que décrit plus haut.

La figure 8 montre comme procède la sélection : l'axe des ordonnées indique les électrodes, l'axe des abscisses la taille du sous-ensemble ; le gris clair indique une électrode conservée, le gris foncé une électrode supprimée. On voit que l'électrode EL11 est la première à être éliminée, suivie par l'EL16 et ainsi de suite. Le sous-ensemble optimal à 3 électrodes, pour l'utilisateur considéré, comprend les électrodes EL3, EL8 et EL15. Si une seule électrode devait être retenue, il s'agirait de l'EL8.

La figure 9 montre comment la valeur maximale du critère de distance varie en fonction de la taille du sous-ensemble d'électrodes. On voit que le passage de 16 à 10 provoque une diminution modérée (de l'ordre de 10%) de cette valeur. En revanche, il serait très pénalisant d'utiliser moins de 3 électrodes.

Ce procédé est simple et robuste. Sa simplicité vient notamment du fait qu'il est immédiat de calculer les matrices de covariance correspondant aux différents sous-ensembles d'électrodes une fois qu'on les a calculées pour l'ensemble complet.

En effet, dans les matrices de covariance, l'information portée par l'électrode i est représentée par la i-ème ligne et la i-ème colonne. Par conséquent, pour déterminer les matrices de covariance correspondant au sous-ensemble de (N-1) électrodes obtenu en enlevant l'électrode i, il suffit de prendre les matrices de covariance de l'ensemble complet de N électrodes et leur enlever la i-ème ligne et la i-ème colonne.

Plus généralement, on peut définir l'opération « red cov » :

$$P \xrightarrow[S]{red\,cov} \widetilde{P}$$

où P est une matrice de covariance, S un vecteur contenant les indices des électrodes à conserver et $\widetilde{P}$ la matrice de covariance réduite. Par exemple si S = [3,7], on aura :

$$P = \begin{bmatrix} p_{1,1} & \cdots & p_{N,1} \\ \vdots & \ddots & \vdots \\ p_{1,N} & \cdots & p_{N,N} \end{bmatrix} \xrightarrow[S=[3,7]]{red\,cov} \widetilde{P} = \begin{bmatrix} p_{3,3} & p_{7,3} \\ p_{3,7} & p_{7,7} \end{bmatrix}$$

Cela signifie que le calcul des matrices de covariance correspondant aux sous-ensembles réduits d'électrodes s'obtient par simple élimination de lignes et de colonnes des matrices de covariance correspondant à l'ensemble complet.

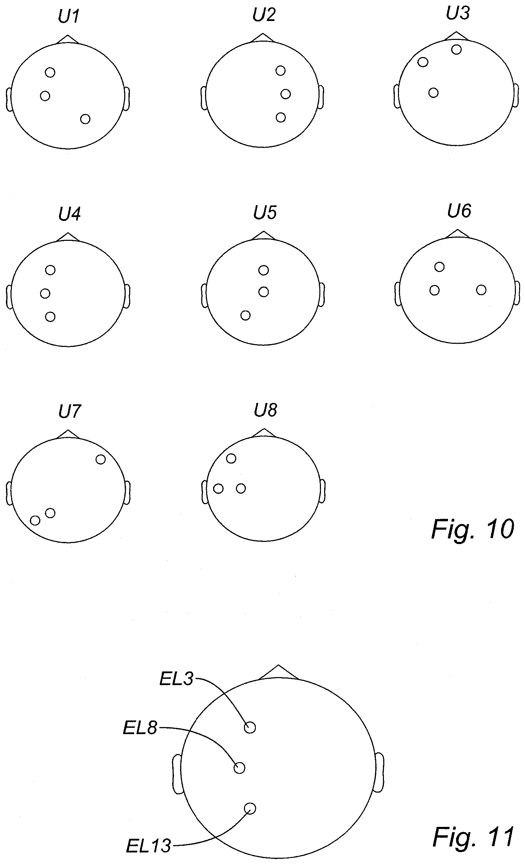

Le procédé que l'on vient de décrire permet une sélection « patient-spécifique » : si on l'applique à plusieurs sujets différents, l'on obtiendra des sous-ensembles « optimaux » d'électrodes différents. C'est ce que montre la figure 10, qui représente les sous-ensembles « optimaux » pour huit utilisateurs U1 - U9.

On peut combiner cette sélection « patient-spécifique » avec une sélection « application-spécifique » : simplement, on sélectionne les électrodes qui apparaissent le plus souvent dans les sous-ensembles optimaux des différents utilisateurs. Ainsi, on peut constater sur la figure 9 que l'électrode EL8 a été sélectionnée pour 5 utilisateurs ; les électrodes EL3 et EL13 ont été sélectionnées pour 3 utilisateurs ; les autres électrodes ont été sélectionnées pour 2, 1 ou 0 utilisateurs. Le sous-ensemble à 3 électrodes optimal, que l'on peut supposer satisfaisant pour la plupart de l'utilisateur, est donc constitué par les électrodes EL3, EL8 et EL13. Ce sous-ensemble est illustré sur la figure 11.

On peut aussi effectuer une sélection ascendante en procédant de la manière suivante. On part d'un ensemble prédéfini d'emplacements possibles pour les électrodes (voir la figure 7). On commence à mettre en place une seule électrode, en essayant tous les emplacements disponibles et en mesurant, pour chaque emplacement, la distance (ou somme des distances) entre les centres de classes. On identifie l'emplacement qui correspond à la distance maximale, et on y laisse l'électrode. A ce point on teste tous les emplacements restants avec une deuxième électrode, et ainsi de suite (on remarquera que, lors de la première itération, la « matrice de covariance » est en réalité un scalaire, ou une « matrice » 1x1). On arrive ainsi à sélectionner un sous-ensemble optimal d'emplacements pour électrodes pour chaque utilisateur. La sélection « application-spécifique » se fait exactement comme pour la sélection descendante (voir les figures 10 et 11).

Il est également possible de combiner, d'une manière connue par elle-même, sélection ascendante et sélection descendante.

## Revendications

**1.** Procédé de commande neuronale directe comportant :

- Une étape de classification de signaux neuronaux ;
- Une étape de génération d'un signal de commande d'un appareil en fonction du résultat de ladite étape de classification ;

l'étape de classification de signaux neuronaux comportant les étapes suivantes :

a. Acquérir, au moyen d'une pluralité d'électrodes (EL1 - EL16) et sur une période déterminée, une pluralité d'échantillons de signaux neuronaux ($x_1(t)$ - $x_{16}(t)$) ;
b. Estimer une matrice de covariance ($X_1$, $X_2$, $X_i$) desdits signaux neuronaux ;
c. Classifier lesdits signaux neuronaux, la classification étant réalisée :

○ soit dans la variété riemannienne des matrices symétriques définies positives de dimensions égales à celles de ladite matrice de covariance,
○ soit dans un espace tangent de ladite variété riemannienne le procédé comportant, avant ladite étape c. de classification :

- La détermination d'un espace tangent de ladite variété riemannienne par rapport à une matrice symétrique positive correspondant à une moyenne géométrique ou à une médiane, dans ladite variété riemannienne, d'un ensemble de matrices de covariance de données d'entrainement associées à des classes ;

- La projection de ladite matrice de covariance à classifier, ainsi que desdites matrices de covariance de données d'entrainement, dans ledit espace tangent ;
- L'application d'un filtrage dimensionnel dans ledit espace tangent ; et
- La rétroprojection des données filtrées dans ladite variété riemannienne pour y effectuer ladite étape c. de classification.

2. Procédé selon la revendication 1, dans lequel ladite classification est réalisée en utilisant un critère basé :

○ Soit sur une distance riemannienne définie sur la variété riemannienne des matrices symétriques définies positives de dimensions égales à celles de ladite matrice de covariance ;
○ Soit sur une distance euclidienne définie sur un espace tangent de ladite variété riemannienne.

3. Procédé selon la revendication 2 dans lequel ledit critère de classification est un critère de moindre distance riemannienne entre ladite matrice de covariance ($P_i$) des signaux à classifier et des matrices symétriques définies positives ($P_1$, $P_2$) représentant des centres des classes.

4. Procédé selon la revendication 2 dans lequel ledit critère de classification est un critère de k plus proches voisins, dans ladite variété riemannienne, entre ladite matrice de covariance des signaux à classifier et des matrices de covariance de données d'entraînement associées à des classes.

5. Procédé selon la revendication 1 ou 2 dans lequel ladite étape c. de classification est mise en oeuvre dans un espace tangent de ladite variété riemannienne, déterminé par rapport à une matrice symétrique positive (P) correspondant à une moyenne géométrique ou à une médiane, dans ladite variété riemannienne, d'un ensemble de matrices de covariance de données d'entrainement associées à des classes.

6. Procédé selon la revendication 5 comportant :

- avant ladite étape c. de classification :

○ La détermination d'un espace tangent de ladite variété riemannienne par rapport à une matrice symétrique positive correspondant à une moyenne géométrique ou à une médiane, dans ladite variété riemannienne, d'un ensemble de matrices de covariance de données d'entrainement associées à des classes ;
○ La projection de ladite matrice de covariance à classifier, ainsi que desdites matrices de covariance des données d'entrainement, dans ledit espace tangent ;
○ L'application d'un filtrage dimensionnel dans ledit espace tangent ; et ensuite :

- La mise en oeuvre de ladite étape c. de classification sur les données filtrées dans ledit espace tangent.

7. Système comportant :

- un ensemble d'électrodes (EL1 - EL16) d'acquisition de signaux neuronaux ; et
- un dispositif de traitement de données (PR) adapté pour recevoir lesdits signaux et les utiliser pour mettre en oeuvre un procédé selon l'une des revendications précédentes.

8. Procédé de sélection d'électrodes d'acquisition de signaux neuronaux pour commande neuronale directe, mis en oeuvre par un dispositif de traitement de données pour recevoir lesdits signaux neuronaux et pour calculer des matrices de covariances représentatives de classes différentes desdits signaux neuronaux et consistant à choisir un sous-ensemble d'électrodes ou d'emplacements pour électrodes parmi un ensemble d'électrodes ou d'emplacements d'électrodes plus vaste, le procédé étant **caractérisé en ce que** ce choix est effectué de manière à maximiser la somme des distances riemanniennes entre des matrices de covariance représentatives de classes différentes de signaux neuronaux issus des électrodes d'un sous-ensemble d'électrodes ou d'emplacements pour électrodes dudit ensemble plus vaste.

9. Procédé de sélection d'électrodes selon la revendication 8 comportant les étapes consistant à :

a. Appliquer à un sujet (U1 - U8) un ensemble initial d'électrodes (EL1 - EL16) d'acquisition de signaux neuronaux correspondant audit ensemble d'électrodes ou d'emplacements d'électrodes plus vaste ;
b. Acquérir, au moyen desdites électrodes et sur une pluralité de périodes déterminées, une pluralité d'échan-

tillons de signaux neuronaux ($x_1(t)$ - $x_{16}(t)$), les signaux correspondant à chaque période étant associés à une parmi une pluralité de classes ;

c. Estimer une matrice de covariance ($X_1$, $X_2$, $X_i$) des signaux neuronaux issus de chaque période, chacune desdites matrices de covariance étant considérée comme un point d'une variété riemannienne ;

d. Pour chacune desdites classes, calculer une matrice symétrique définie positive représentant un centre de classe ; et

e. Sélectionner un sous-ensemble desdites électrodes de manière à maximiser la somme des distances riemanniennes entre des matrices symétriques définies positives représentant des centres desdites classes pour un sous-ensemble desdites électrodes, ces matrices étant dérivées des matrices calculées à l'étape d.

10. Procédé selon la revendication 9 dans lequel ladite étape e. est mise en oeuvre de manière itérative et comporte, à chaque itération, l'élimination d'une électrode choisie de manière à minimiser la diminution de distance riemannienne entre les matrices de covariance moyennes ou médianes desdites classes qui en résulte.

11. Procédé de sélection d'électrodes selon la revendication 8 comportant les étapes consistant à :

a. Déterminer un ensemble d'emplacements pour l'application d'électrodes d'acquisition de signaux neuronaux sur un sujet ;

b. Introduire progressivement des électrodes de manière à incrémenter de manière maximale ladite somme des distances riemanniennes entre des matrices de covariance représentatives de classes différentes de signaux issus desdites électrodes.

12. Procédé selon l'une des revendications 8 à 11 dans lequel chaque classe de signaux neuronaux correspond à une action imaginée par le sujet.

13. Procédé selon l'une des revendications 8 à 12 dans lequel chacune desdites électrodes est adaptée pour acquérir des signaux neuronaux générés par une région déterminée du cerveau du sujet.

14. Procédé de sélection d'électrodes d'acquisition de signaux neuronaux pour commande neuronale directe comportant :

- la mise en oeuvre d'un procédé selon l'une des revendications 8 à 13 pour une pluralité de sujets (U1 - U8), avec un même ensemble initial d'électrodes ou d'emplacement pour électrodes pour tous les sujets, de manière à déterminer un sous-ensemble d'électrodes pour chaque sujet ;

- la sélection des électrodes ou des emplacements pour électrodes (EL3, EL8, EL13) dudit ensemble initial qui appartiennent au plus grand nombre desdits sous-ensembles.

15. Procédé de commande neuronale directe selon la revendication 1, comportant une opération préalable de sélection des électrodes d'acquisition de signaux neuronaux selon l'une des revendications 8 à 14.

**Patentansprüche**

1. Direktes neuronales Steuerverfahren, das Folgendes aufweist:

- Einen Schritt zur Klassifizierung von neuronalen Signalen;

- Einen Schritt zur Erzeugung eines Steuersignals eines Geräts in Abhängigkeit vom Ergebnis des Klassifizierungsschritts;

wobei der Schritt der Klassifizierung von neuronalen Signalen die folgenden Schritte aufweist:

a. Erfassen, mittels einer Vielzahl von Elektroden (EL1 - EL16) und über einen bestimmten Zeitraum, einer Vielzahl von neuronalen Signalabtastungen ($x_1(t)$ - $x_{16}(t)$);

b. Schätzen einer Kovarianzmatrix ($X_1$, $X_2$, $X_i$) der neuronalen Signale;

c. Klassifizieren der neuronalen Signale, wobei die Klassifizierung durchgeführt wird:

- entweder in der riemannschen Mannigfaltigkeit positiv definierter symmetrischer Matrizen mit Dimensionen, die gleich denjenigen der Kovarianzmatrix sind,

- oder in einem Tangentialraum der riemannschen Mannigfaltigkeit, wobei das Verfahren vor dem Schritt c. der Klassifizierung Folgendes aufweist:

- Die Bestimmung eines Tangentialraums der riemannschen Mannigfaltigkeit in Bezug auf eine positiv symmetrische Matrix, die einem geometrischen Mittel oder einem Median entspricht, in der riemannschen Mannigfaltigkeit, eines Satzes von Kovarianzmatrizen von den Klassen zugeordneten Trainingsdaten;
- Die Projektion der zu klassifizierenden Kovarianzmatrix sowie der Kovarianzmatrizen Trainingsdaten in den Tangentialraum;
- Die Anwendung einer dimensionalen Filterung in dem Tangentialraum; und
- Die Rückprojektion der gefilterten Daten in die riemannsche Mannigfaltigkeit, um darin den Schritt c. der Klassifizierung durchzuführen.

2. Verfahren nach Anspruch 1, wobei die Klassifizierung unter Verwendung eines Kriteriums durchgeführt wird, basierend auf:

- Entweder auf einem riemannschen Abstand, der auf der riemannschen Mannigfaltigkeit von positiv definierten symmetrischen Matrizen mit Dimensionen definiert ist, die gleich denjenigen der Kovarianzmatrix sind;
- Oder auf einem euklidischen Abstand, der auf einem Tangentialraum der riemannschen Mannigfaltigkeit definiert ist.

3. Verfahren nach Anspruch 2 wobei das Klassifizierungskriterium ein Kriterium des geringsten riemannschen Abstands zwischen der Kovarianzmatrix ($P_i$) der zu klassifizierenden Signale und positiv definierten symmetrischen Matrizen ($P_1$, $P_2$), die Zentren der Klassen darstellen, ist.

4. Verfahren nach Anspruch 2 wobei das Klassifizierungskriterium ein Kriterium der k nächstliegenden Nachbarn in der riemannschen Mannigfaltigkeit zwischen der Kovarianzmatrix der zu klassifizierenden Signale und Kovarianzmatrizen von Trainingsdaten, die den Klassen zugeordnet sind, ist.

5. Verfahren nach Anspruch 1 oder 2, wobei der Schritt c. der Klassifizierung in einem Tangentialraum der riemannschen Mannigfaltigkeit implementiert wird, der in Bezug auf eine positiv symmetrische Matrix (P) bestimmt wird, die einem geometrischen Mittel oder einem Median in der riemannschen Mannigfaltigkeit eines Satzes von Kovarianzmatrizen von den Klassen zugeordneten Trainingsdaten entspricht.

6. Verfahren nach Anspruch 5, das Folgendes aufweist:

- vor dem Schritt c. der Klassifizierung:

- Die Bestimmung eines Tangentialraums der riemannschen Mannigfaltigkeit in Bezug auf eine positiv symmetrische Matrix, die einem geometrischen Mittel oder einem Median entspricht, in der riemannschen Mannigfaltigkeit, eines Satzes von Kovarianzmatrizen von den Klassen zugeordneten Trainingsdaten;
- Die Projektion der zu klassifizierenden Kovarianzmatrix sowie der Kovarianzmatrizen der Trainingsdaten in den Tangentialraum;
- Die Anwendung einer dimensionalen Filterung in dem Tangentialraum; und dann:

- Die Implementierung des Schritts c. der Klassifizierung auf die gefilterten Daten in dem Tangentialraum.

7. System, das Folgendes aufweist:

- einen Satz von Elektroden (EL1 - EL16) zur Erfassung neuronaler Signale; und
- eine Datenverarbeitungsvorrichtung (PR), die dazu angepasst ist, die Signale zu empfangen und sie zu verwenden, um ein Verfahren nach einem der vorstehenden Ansprüche zu implementieren.

8. Verfahren zur Auswahl von Elektroden zur Erfassung neuronaler Signale für die direkte neuronale Steuerung, implementiert durch eine Datenverarbeitungsvorrichtung zum Empfangen der neuronalen Signale und zum Berechnen von Kovarianzmatrizen, die für unterschiedliche Klassen der neuronalen Signale repräsentativ sind, und bestehend aus dem Auswählen eines Teilsatzes von Elektroden oder Elektrodenpositionen aus einem größeren Satz von Elektroden oder Elektrodenpositionen, wobei das Verfahren **dadurch gekennzeichnet ist, dass** diese Auswahl derart erfolgt, dass die Summe der riemannschen Abstände zwischen Kovarianzmatrizen, die unterschiedliche Klassen von neuronalen Signalen darstellen, die von den Elektroden eines Teilsatzes von Elektroden oder Elektrodenpositionen des größeren Satzes stammen, maximiert wird.

9. Elektrodenauswahlverfahren nach Anspruch 8, das die Schritte aufweist, bestehend aus dem:

   a. Anwenden eines anfänglichen Elektrodensatzes (EL1 - EL16) auf ein Subjekt (U1 - U8) zur Erfassung neuronaler Signale, die dem Elektrodensatz oder größeren Elektrodenpositionen entsprechen;
   b. Erfassen, mittels der Elektroden und über eine Vielzahl von bestimmten Perioden, einer Vielzahl von neuronalen Signalabtastungen ($x_1(t)$ - $x_{16}(t)$), wobei die Signale, die jeder Periode entsprechen, einer aus einer Vielzahl von Klassen zugeordnet sind;
   c. Schätzen einer Kovarianzmatrix ($X_1$, $X_2$, $X_i$) der aus jeder Periode stammenden neuronalen Signale, wobei jede der Kovarianzmatrizen als ein Punkt einer riemannschen Mannigfaltigkeit betrachtet wird;
   d. Berechnen einer positiv definierten symmetrischen Matrix, die ein Klassenzentrum darstellt, für jede der Klassen; und
   e. Auswählen eines Teilsatzes der Elektroden, um die Summe der riemannschen Abstände zwischen positiven definierten symmetrischen Matrizen, die Zentren der Klassen darstellen, für einen Teilsatz der Elektroden zu maximieren, wobei diese Matrizen von den in Schritt d berechneten Matrizen abgeleitet werden.

10. Verfahren nach Anspruch 9, wobei der Schritt e. iterativ implementiert wird und bei jeder Iteration die Eliminierung einer Elektrode aufweist, die derart ausgewählt ist, dass die daraus resultierende Abnahme des riemannschen Abstands zwischen den mittleren oder medianen Kovarianzmatrizen der Klassen minimiert wird.

11. Elektrodenauswahlverfahren nach Anspruch 8, das die Schritte aufweist, bestehend aus dem:

   a. Bestimmen eines Satzes von Positionen für die Anbringung von Elektroden zur Erfassung neuronaler Signale an einem Subjekt;
   b. Progressives Einführen von Elektroden, so dass die Summe der riemannschen Abstände zwischen Kovarianzmatrizen, die unterschiedliche Klassen von aus den Elektroden stammenden Signalen darstellen, maximal inkrementiert wird.

12. Verfahren nach einem der Ansprüche 8 bis 11, wobei jede Klasse von neuronalen Signalen einer vom Subjekt imaginierten Handlung entspricht.

13. Verfahren nach einem der Ansprüche 8 bis 12, wobei jede der Elektroden angepasst ist, um neuronale Signale zu erfassen, die von einer bestimmten Region des Gehirns des Subjekts erzeugt werden.

14. Verfahren zur Auswahl von Elektroden zur Erfassung von neuronalen Signalen für die direkte neuronale Steuerung, das Folgendes aufweist:

   - Implementierung eines Verfahrens nach einem der Ansprüche 8 bis 13 für eine Vielzahl von Subjekten (U1 - U8), mit einem gleichen anfänglichen Satz von Elektroden oder Elektrodenpositionen für alle Subjekte, um einen Teilsatz von Elektroden für jedes Subjekt zu bestimmen;
   - die Auswahl der Elektroden oder Elektrodenpositionen (EL3, EL8, EL13) des anfänglichen Satzes, die zu der größten Anzahl der Teilsätze gehören.

15. Direktes neuronales Steuerverfahren nach Anspruch 1, das einen vorherigen Vorgang der Auswahl von Elektroden zur Erfassung neuronaler Signale nach einem der Ansprüche 8 bis 14 aufweist.

## Claims

1. A direct neural control method comprising:

   - a step of classifying neural signals in accordance with any preceding claim;
   - a step of generating a signal for controlling an apparatus

as a function of the result of said classification step the step of classification comprising the following steps:

> a) acquiring a plurality of neural signal samples ($x_1(t)$ - $x_{16}(t)$) over a determined period of time using a plurality of electrodes (EL1 - EL16);
> b) estimating a covariance matrix ($X_1$, $X_2$, $X_i$) of said neural signals; and
> c) classifying said neural signals, the classification being performed:
>
>> · either in the Riemann manifold of symmetric positive definite matrices of dimensions equal to the dimensions of said covariance matrix;
>> · or else in a tangent space of said Riemann manifold the method including, prior to said classification step c):
>>
>>> - determining a tangent space of said Riemann manifold relative to a symmetric positive matrix corresponding to a geometric mean or a median in said Riemann manifold of a set of covariance matrices of training data associated with classes;
>>> - projecting said covariance matrix that needs to be classified together with said covariance matrices of training data into said tangent space;
>>> - applying dimensional filtering in said tangent space; and
>>> - back-projecting the filtered data into said Riemann manifold in order to perform said classification step c).

2. A method according to claim 1, wherein said classification is performed using a criterion based:

> · either on a Riemann distance defined on the Riemann manifold of symmetric positive definite matrices of dimensions equal to the dimensions of said covariance matrix; or else
> · on a Euclidean distance defined on a tangent space of said Riemann manifold.

3. A method according to claim 2, wherein said classification criterion is a criterion of least Riemann distance between said covariance matrix ($P_i$) of the signals to be classified and the symmetric positive definite matrices ($P_1$, $P_2$) representing centers of the classes.

4. A method according to claim 2, wherein said classification criterion is a k nearest neighbors criterion in said Riemann manifold, between said covariance matrix of the signals that are to be classified and the covariance matrices of training data associated with the classes.

5. A method according to claim 1 or claim 2, wherein said classification step c) is implemented in a tangent space of said Riemann manifold, determined relative to a symmetric positive matrix (P) corresponding to a geometric mean or to a median in said Riemann manifold of a set of covariance matrices of training data associated with classes.

6. A method according to claim 5, comprising:

> · prior to said classification step c):
>
>> · determining a tangent space of said Riemann manifold relative to a symmetric positive matrix corresponding to a geometric mean or to a median in said Riemann manifold of a set of covariance matrices of training data associated with classes;
>> · projecting said covariance matrix that is to be classified together with said covariance matrices of training data into said tangent space; and
>> · applying dimensional filtering in said tangent space; and then
>
> · implementing said classification step c) on the filtered data in said tangent space.

7. A system comprising:

> · a set of neural signal acquisition electrodes (EL1 - EL16); and
> · a data processor device (PR) adapted to receive said signals and to use them for implementing a method according to any preceding claim.

8. An electrode selection method for selecting neural signal acquisition electrodes for direct neural control, the method being implemented by a data processing device for receiving said neural signals and for calculating covariance matrices representative of different classes of said neural signals and consisting in selecting a subset of electrodes or electrode locations from a set having a greater number thereof or larger electrode locations, the method being **characterized in that** this selection is made so as to maximize the sum of the Riemann distances between covariance matrices representative of signals of different classes coming from said electrodes of a subset of electrodes or electrode locations from said set having a greater number thereof.

9. An electrode selection method according to claim 8, the method comprising the steps consisting in:

    a) applying an initial set of neural signal acquisition electrodes (EL1 - EL16) corresponding to the set of electrodes or to the larger electrode locations to a subject (U1 - U8);
    b) using said electrodes and a plurality of determined periods to acquire a plurality of neural signal samples ($x_1(t)$ - $x_{16}(t)$), the signals corresponding to each period being associated with one out of a plurality of classes;
    c) estimating a covariance matrix ($X_1$, $X_2$, $X_i$) of the neural signals from each period, each of said covariance matrices being considered as a point of a Riemann manifold;
    d) for each of said classes, calculating a symmetric positive definite matrix representing a class center; and
    e) selecting a subset of said electrodes in such a manner as to maximize the sum of the Riemann distances between said symmetrical positive-definite matrices, representing centers of said classes, corresponding to a subset of said electrodes, these matrices being derived from the matrices calculated in step d.

10. A method according to claim 15 or claim 9, wherein said step e) is implemented iteratively and on each iteration includes eliminating an electrode that is selected in such a manner as to minimize the decrease in the Riemann distance between the mean or median covariance matrices of said classes that results therefrom.

11. An electrode selection method according to claim 8, including the steps consisting in:

    a. determining a set of locations for applying neural signal acquisition electrodes on a subject; and
    b. progressively applying electrodes so as to maximally increment some sum of the Riemann distances between the covariance matrices representative of different classes of signals from said electrodes.

12. A method according to any one of claims 8 to 11, wherein each class of neural signals corresponds to an action imagined by the subject.

13. A method according to any one of claims 8 to 12, wherein each of said electrodes is adapted to acquire neural signals generated by a determined region of the subject's brain.

14. A method of selecting neural signal acquisition electrodes for direct neural control, the method comprising:

    - implementing a method according to any one of claims 8 to 13 on a plurality of subjects (U1 - U8) with the same initial set of electrodes or locations for electrodes for all of the subjects, so as to determine a subset of electrodes for each subject; and
    - selecting electrodes (EL3, EL8, EL13) of said initial set that belong to the greatest number of said subsets.

15. A direct neural control method according to claim 1, including a prior operation of selecting neural signal acquisition electrodes in accordance with any one of claims 8 to 14.

Fig. 1

Fig. 2

**Fig. 5**

**Fig. 6**

Fig. 7

Fig. 8

Taille du sous ensemble ($N_1$)

Fig. 9

Valeur maximale du critère de distance

Taille du sous ensemble ($N_1$)

U1

U2

U3

U4

U5

U6

U7

U8

Fig. 10

EL3

EL8

EL13

Fig. 11

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 20080063285 A

- US 2008063264 A1

**Littérature non-brevet citée dans la description**

- **F. LOTTE ; M. CONGEDO ; A. LÉCUYER ; F. LAMARCHE ; B. ARNALDI.** A review of classification algorithms for EEG-based brain-computer interfaces. *Journal of Neural Engineering,* 2007, vol. 4
- **A. BARACHANT ; T. AKSENOVA ; S. BONNET.** Filtrage spatial robuste à partir d'un sous-ensemble optimal d'électrodes en BCI EEG. *GRETSI 2009,* 08 Août 2009
- **W. GERSCH et al.** A Kullback Leibler-nearest neighbor rule classification of EEGs : The EEG population screening problem, an anesthésia level EEG classification application. *Computers and Biomedical Research,* Juin 1980, vol. 13 (3), 283-296
- **B. KAMOUSI et al.** « Classification of motor imagery by means of cortical current density estimation and Von Neumann entropy. *Journal of Neural Engineering,* Juin 2007, vol. 4 (2), 17-25

- **YILI LI et al.** EEG signal classification based on a Riemannian distance measure. *Proceedings of 2009 IEEE Toronto International Conférence Science and Technology for Humanity (TIC-STH 2009),* 26 Septembre 2009, 268-273
- **F. BARABARESCO et al.** Espace Riemannien symétrique et géométrie des espaces de matrices de covariance: équations de diffusion et calculs de médianes. *Actes du Colloque GRETSI,* 08 Septembre 2009, vol. 2009
- **C. SANNELLI et al.** « On optimal channe! configuration for SMR-based Brain-Computer interfaces. *Brain Topography,* 17 Février 2010, vol. 23 (2), 186-193
- **R. O. DUDA ; P. E. HART ; D.G. STORK.** Pattern Classification. J. Wiley & Sons Inc, 2000, 117-124